# EUROPEAN PATENT APPLICATION

(11) **EP 3 524 207 A1**
(43) Date of publication of application: **14.08.2019**
(21) Application number: 19156980.5
(22) Date of filing: 13.02.2019
(51) Int. Cl.: A61F 2/07

(54) **DOUBLE RADIOPAQUE MARKERS ON AN ENDOVASCULAR STENT**

(30) Priority: 13.02.2018 US 201815895437
(71) Applicant: Cook Medical Technologies LLC, Bloomington, IN 47404 (US)
(72) Inventor: WOONG, Kim, West Lafayette, IN Indiana 47906 (US)
(74) Representative: Warren, Caroline Elisabeth

(57) **Abstract**

A stent graft device having radiopaque markers for rotational orientation. The stent graft device may include an expandable stent frame and attached graft member. Two radiopaque markers, one the mirror image of the other, positioned on opposite sides of the stent graft at the same axial position along the longitudinal axis of the stent graft device, may be used to provide fine granularity rotational orientation of the stent graft. The two radiopaque markers may be attached to a same stent element and be in the form of checkmarks or some other linear asymmetric design that allow a user to view rotational orientation of the stent graft device by the amount of alignment and overlap of the two radiopaque markers when viewed via an imaging device such as an x-ray.

## Description

### BACKGROUND

The present disclosure relates generally to apparatuses and methods for treating vascular conditions, and more specifically, to apparatuses and methods for aiding alignment of a medical device in a vessel.

An aortic aneurysm is a disease condition in which the aorta (the large artery coming off the left side of the heart) is abnormally dilated. Because aortic aneurysms can rupture and be fatal, either surgical or endovascular approaches may be required for treatment. Endovascular approaches are less invasive and thus often preferred over surgical approaches. Endovascular approaches usually involve the placement of a covered stent graft in a preferred orientation inside the aneurysm to maintain blood flow through the aorta while diverting blood away from the aneurysm.

An X-ray is usually the mode of endovascular visualization and there can be challenges with seeing and orienting a stent graft or other medical implement with an X-ray device during an endovascular procedure.

### BRIEF SUMMARY

In order to address the challenges of visualizing and orienting a stent graft or other medical implement during an endovascular procedure, a system and method for providing improved visualization and orientation under an imaging system such as an X-ray is provided.

Aspects of the invention are set out in the independent claims, which define a number of alternative solutions to the challenges highlighted above. Preferred features are set out in the dependent claims.

According to one aspect, a stent graft device is provided that includes a stent frame having a central axis and a generally tubular graft member attached to the stent frame, where the stent graft device has a compressed state and an expanded state, and where a diameter of the stent graft in the expanded state is greater than that of the stent graft device in the compressed state. A first radiopaque marker is positioned on a first longitudinally extending side of the stent graft device and a second radiopaque marker is positioned on a second longitudinally extending side of the stent graft device that is on an opposite side of the stent graft device from the first longitudinally extending side. The second radiopaque marker may be positioned at a same axial position along the central axis of the stent graft device as the first radiopaque marker, and the second radiopaque marker, as viewed from the second longitudinally extending side, is a mirror image of the first radiopaque marker as viewed from the first longitudinally extending side. With this arrangement, a unique rotational position of the stent graft device is detectable under x-ray or fluoroscopy imaging via a spacing, which may be a partial or complete eclipsing, of the first and second radiopaque markers in an image of the stent graft device.

According to another aspect, a stent graft device includes a plurality of stent frame elements arranged along a central axis with a graft member attached with the stent frame elements. The stent graft device may have a compressed state and an expanded state, where a diameter of the stent graft device in the expanded state is greater than that of the stent graft device in the compressed state. A first radiopaque marker having an asymmetric linear shape may be positioned along a circumference of the stent graft device at a 180 degree circumferential offset from a second radiopaque marker comprising a mirror image of the asymmetric linear shape of the first radiopaque marker. The first and second radiopaque markers may be positioned on the stent graft device at a same axial location along the central axis. A unique rotational position of the stent graft device is detectable via a spacing, which may be a partial or complete eclipsing, of the first and second radiopaque markers in an image of the stent graft device.

In yet another aspect, a stent graft device is disclosed with a radially expandable stent frame having a central axis and a tubular graft member attached with a surface of the stent frame, where the stent graft device has a compressed state and an expanded state, and where a diameter of the stent graft device in the expanded state is greater than that of the stent graft device in the compressed state. First and second radiopaque markers each a comprising a linear pattern having at least one bend are attached to the stent graft device. The first radiopaque marker is fixedly positioned on a first side of the stent graft device and the second radiopaque marker is fixedly positioned on a second side of the stent graft device on an opposite side of the stent graft device from the first side, and at a same axial position along the stent graft device as the first radiopaque marker. An orientation of the first radiopaque marker when the first radiopaque marker is viewed from the first side of the stent graft device is a mirror image of an orientation of the second radiopaque marker when the second radiopaque marker viewed from the second side of the stent graft device. Additionally, the orientation of the first radiopaque marker is a same orientation as the orientation of the second radiopaque marker in an image generated by an imaging system through the stent graft device. A unique rotational position of the stent graft device is detectable via a location of the first and second radiopaque markers in the image generated by the imaging system.

### BRIEF DESCRIPTION OF THE DRAWINGS

FIG. 1A is a perspective view of one embodiment of the front side of an uncompressed stent graft with a first alignment marker arranged as described herein.
FIG. 1B is a perspective view of the stent graft of FIG. 1B in a compressed state.
FIG. 1C is an end view of the stent graft taken along line 1C-1C of FIG. 1A showing the relative positioning of first and second radiopaque markers on the front side and the back side, respectively, of the stent graft.
FIG. 1D is a perspective view of the back side of the uncompressed stent graft of FIG. 1A with a second alignment marker arranged as described herein.
FIG. 1E is a perspective view of the stent graft of FIG. 1D in a compressed state.
FIG. 1F is a perspective sectional view of the stent graft of FIG. 1D.
FIG. 2A is a perspective view of an x-ray projection of the stent graft of FIG. 1A showing the stent graft of FIG. 1A rotated -5 degrees away from a desired alignment, where the first and second radiopaque markers show a measurable relative offset.
FIG. 2B is the x-ray projection of the stent graft of FIG. 1A when the stent graft is in a desirable rotational orientation offset of 0 degrees where the first radiopaque marker substantially eclipses the second radiopaque marker on the opposite side of the stent graft.
FIG. 2C is the x-ray projection of the stent graft of FIG. 1A showing the stent graft of FIG. 1A rotated +5 degrees away from a desired alignment.
FIGS. 3A-3B illustrate an enlarged sectional view of the radiopaque marker of FIGS. 1E and 1D, respectively.
FIGS. 4A-4B illustrate and enlarged sectional view of the radiopaque marker of FIGS. 1B and 1A, respectively.
FIG. 5A illustrates a thoracoabdominal aortic aneurism (TAAA) stent graft with four directional branches.
FIG. 5B is a sectional perspective view of the TAAA stent graft of FIG. 5A.

### DETAILED DESCRIPTION

Stent graft devices are used to treat abdominal aortic aneurysms by reinforcing the wall of the aorta to prevent a weakened area from rupturing. Different types of stent graft devices, such as thoracoabdominal aortic aneurism (TAAA) devices, often use radiopaque markers to permit improved visualization of the position, including rotational orientation, of the stent graft during insertion and placement in a body. Rather than relying on orientation mechanisms outside of the stent graft device for position and orientation, for example on an insertion sheath used to introduce a stent graft device into a body, the embodiments below describe a stent graft device with radiopaque markers strategically shaped and positioned on the stent graft device itself to assist with rotational orientation detection.

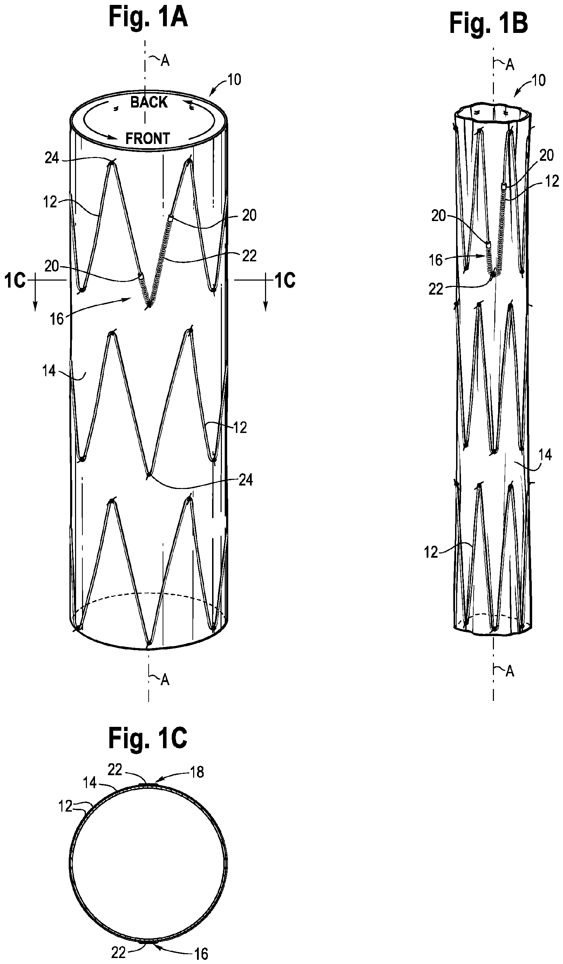

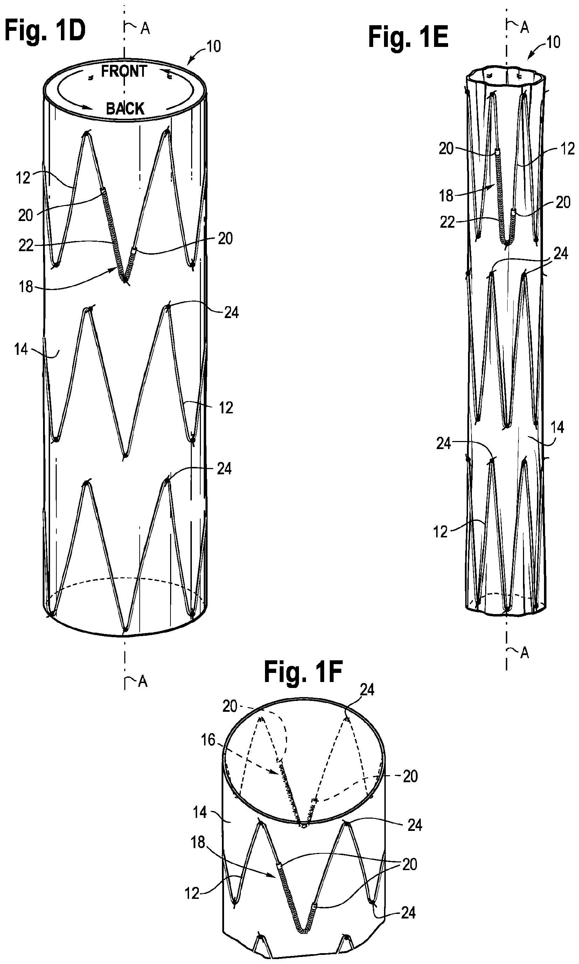

Referring now to FIGS. 1A-1F, an embodiment of stent graft 10 having a radiopaque alignment system for use in aligning and positioning the stent graft in a vessel is illustrated. The stent graft 10 is shown in an expanded, also referred to as deployed, position in FIG. 1A with a first radiopaque marker 16 facing forward on a front side, also referred to herein as the first longitudinally extending side. FIG. 1B illustrates that same stent graft 10 rotated 180 degrees about its longitudinal axis A. A second radiopaque marker 18, one that is a mirror image of the first radiopaque marker 16, is positioned on the back side, also referred to herein and the second longitudinally extending side, of the stent graft 10 and at the same axial position along longitudinal axis A as the first radiopaque marker 16. As shown in FIG. 1C, the first and second radiopaque markers 16, 18 are aligned at opposite sides of the circumference of the stent graft 10 in 12 o'clock and 6 o'clock (180 degree circumferential offset) positions, respectively. FIGS. 1D and 1E show the stent graft 10 rotated such that the second radiopaque marker 18 is facing front, where FIG. 1D illustrates the expanded state and FIG. 1E illustrates the compressed state. FIG. 1F provides a perspective view of the stent graft 10 illustrating an orientation and relative positioning of the radiopaque markers 16, 18 on opposite sides of the stent graft. As noted above, the orientation of the first and second radiopaque markers are mirror images of one another as viewed from the outside of the stent graft 10, and are thus oriented in a same direction when viewed on an image generated with an imaging device, such as a fluoroscopic imaging device, that provides an image through the stent graft 10,

The stent graft 10 may include one or more sets of expandable stent frame elements 12 aligned along the longitudinal axis A that together define a stent frame. The inner portion of each stent frame element 12 facing the interior of the stent graft device 10 may also be referred to herein as the luminal surface and the exterior portion of each stent frame element 12 may also be referred to herein as the abluminal surface of the stent frame element 12. Although shown in FIGS. 1A-1F as positioned on the outside of the tubular graft portion 14, each of the stent frame elements 12 may be positioned inside or on the tubular graft portion 14 utilizing any of a number of known stent wire and graft materials, and attached together by stitching 24 to form the stent graft 10. As shown in FIGS 1A-1B, the stent frame elements 12 may be mounted on the outside of the tubular graft portion 14 and may be attached to the tubular graft portion 14 by stitching 24 at various locations along the wire of the stent frame element 12.

The first and second radiopaque markers 16, 18 may be attached to, or form part of a stent frame element 12. In one implementation, the first and second radiopaque markers 16, 18 may be in the form of a checkmark or j-shape, with the second radiopaque marker 18 being the mirror image of the first radiopaque marker 16 when each is separately viewed by an observer looking directly at the respective marker from the side of the stent graft 10 that the marker is located. The first and second radiopaque markers 16, 18 may be formed in any of a number of ways. In the example of FIGS. 1A-1E, the radiopaque markers are formed by winding a radiopaque metal wire 22 around respective bends in a stent frame element 12 and attaching a band 20 of the radiopaque metal at the ends of the wire 22 to keep the wire in place. In different embodiments, the radiopaque markers 16, 18 need not be made up of the specific combination of the coiled wire 22 and the bands 20 shown. Instead, the radiopaque markers may be made up only of a coiled wire held in place by an adhesive or sutures on the stent, or may only be made up of a series of multiple bands that are spaced along a stent frame element without the addition of a coiled wire. Different combinations of the radiopaque materials may be implemented to achieve the linear-shaped radiopaque markers. Any of a number of materials that are readily visible under an x-ray scan (i.e., radiopaque materials), for example metals such as gold or platinum, may be used for the radiopaque markers 16, 18. Although shown as wound around and attached to the stent frame elements in the example of FIGS. 1A-1F, in other implementations the radiopaque markers 16, 18 may be attached to the stent graft 10 (to either or both of the stent frame elements 12 or tubular graft portion 14) through welding, winding, weaving, printing, adhering or any of a number of known mechanisms for attaching materials to a stent graft.

When the stent graft 10 is in the contracted, or undeployed position (see FIGS. 1B and 1E), the markers 16, 18 are still positioned at 180 degrees from each other but are circumferentially spaced closer to one another due to the smaller overall circumference of the stent graft 10. When the stent frame element 12 is in the contracted position shown in FIGS 1B and 1E, for example, the radiopaque markers 16, 18 may take on more of a j-shape due to the bending of the stent frame element 12 and the added wire material wound around the angled bend in the stent frame element 12. The radiopaque markers 16, 18 are preferably still aligned at opposite sides of the stent frame element 12, but at the same axial location with respect to the central axis (A), when the stent graft is in the compressed position so that visual alignment via a fluoroscope is available when the stent graft 10 is deployed or undeployed.

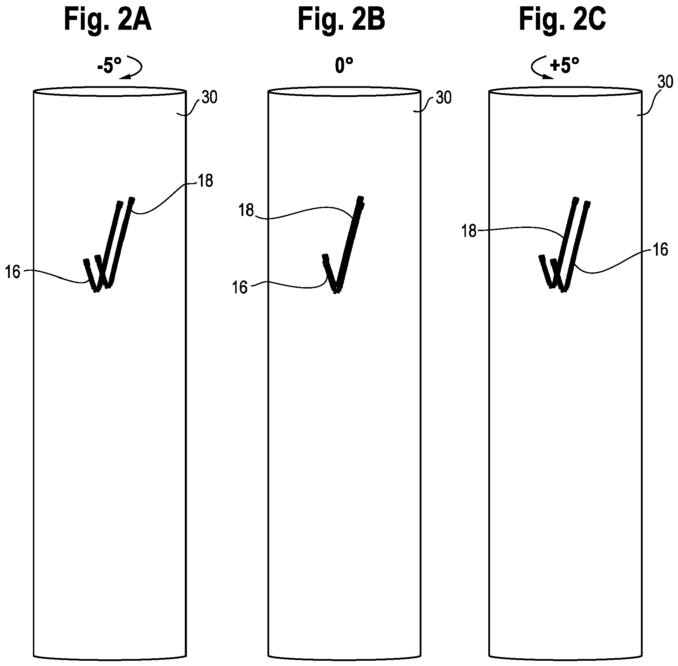

FIGS. 2A-2C show hypothetical x-ray projections 30 of the uncompressed (deployed) stent graft 10 of FIGS. 1A and 1D in different rotational orientations about the longitudinal axis A of the stent graft 10. More specifically, FIGS. 2A-2C illustrate the fine granularity of different possible overlaps of the check-shaped radiopaque markers 16, 18 that are possible. FIG. 2A illustrates a hypothetical 5 degree clockwise rotation away from alignment, where the first radiopaque marker on the surface of the stent graft facing the observer has appears slightly to the left of the second radiopaque marker 18 on the back of the stent graft 10. FIG. 2B illustrates a hypothetical 0 degree offset of the first and second radiopaque markers where the first radiopaque marker appears to fully eclipse and align with the second radiopaque marker. FIG. 2C illustrates a hypothetical 5 degree offset in the counterclockwise direction around the longitudinal axis A where the image of the first radiopaque marker 16 appears slightly to the right of the image of the second radiopaque marker 18 on the back side of the stent graft.

The mirror image orientation of the first and second radiopaque markers 16, 18, as viewed from the respective facing sides of the stent graft, permit the observer to see the first and second radiopaque markers as facing the same direction when viewed via a fluoroscopic device (i.e. when the second radiopaque marker 18 is seen via x-ray looking through the stent graft 10 from the side that the first radiopaque marker is mounted on). Also a relatively thin or linear pattern, such as the mirror image j-shape or checkmark shape example in FIGS. 1A-1E, may assist with providing a finer, higher resolution overlap as viewed with the imaging device than a thicker shape. With a thinner width profile that a linear pattern presents, a smaller amount of one radiopaque marker overlaps or eclipses the other radiopaque marker when the stent graft 10 is imaged and it may be easier to visualize finer increments of rotation as compared to larger two-dimensional shapes.

Assuming the hypothetical X-ray projection 30 of the uncompressed stent graft 10 in FIG. 2B is shown in the "correct" orientation in which the stent graft 10 is intended to be positioned during deployment, then the user seeing hypothetical offset images such as FIGS. 2A and 2C can rotate the stent graft to achieve the desired orientation. Thus, the 0° alignment of FIG. 2B shows one of the radiopaque markers substantially eclipsing the other, where the asymmetry of the radiopaque markers 16, 18 also allows for a visual determination of whether the front or back of the stent graft 10 is being viewed. Each of the orientations of the stent graft 10 other than what is shown in FIG. 2B would be seen as discernible offsets (here hypothetical images of +/- 5 ° alignments are illustrated in FIGS. 2A and 2C) when viewed via x-ray and would thus provide feedback to the user that adjustments may be needed for proper positioning. The assumption in the above discussion is that the orientation of FIG. 2B is the desired orientation, however any other orientation of the first and second radiopaque markers 16, 18 that are positioned at the same axial, and opposite circumferential, positions may be preselected as the correct orientation in different applications. Thus, it should be noted that in other embodiments, the "correct" orientation could be the hypothetical projection of FIGS. 2A or 2C, or another projection with all other offsets seen as incorrect projections.

When inserting the stent graft 10 into a vessel in a body, a medical professional may look for a predefined, unique alignment of the first and second radiopaque markers 16, 18, such as shown in FIGS. 2A-2C, as the compressed stent graft is being maneuvered into position in the vessel. When the desired visibly identifiable alignment of the first and second markers 16, 18 is attained, then the stent graft 10 may be expanded and fixed in place in the vessel.

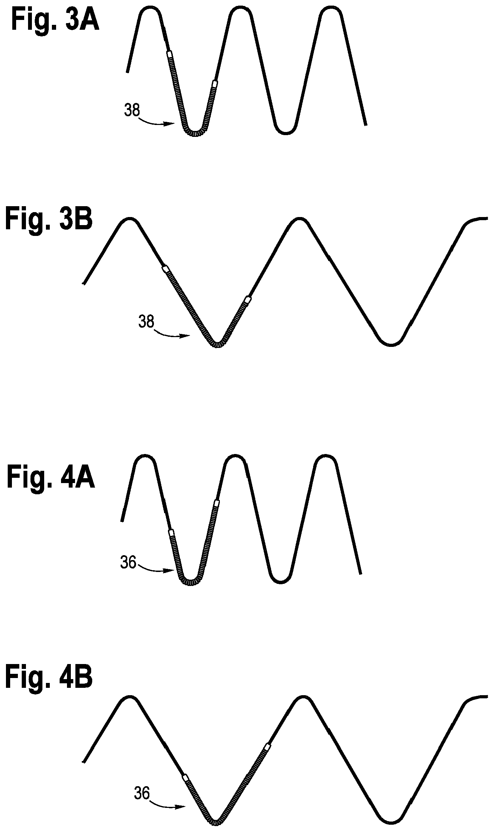

Referring to FIGS. 3A -3B the second radiopaque marker 38 (corresponding to second radiopaque marker 18 in FIGS 1-2) is shown in enlarged form both in more of a j-shape orientation (FIG. 3A) and in a checkmark shape orientation (FIG. 3B). The second radiopaque marker is shown as would be viewed by an observer looking at the stent graft device 10 from the side of the stent graft device that the second marker is mounted on. Similarly, the first radiopaque marker 36 (corresponding to first radiopaque marker 16 in FIGS 1-2) is shown in a j-shape orientation (FIG. 4A) and in a checkmark shape orientation (FIG. 4B). Although a j-shaped or checkmark shaped pattern is shown, any of a number of other linear patterns may be used where a mirror image of a first radiopaque marker is used as the second radiopaque marker, such that an image taken using an imaging technique such as fluoroscopy would show the first and second radiopaque markers on opposite sides of the stent graft device circumference in a same orientation. With this arrangement of linear patterns, the smaller width of the linear portions of the radiopaque markers may permit for higher resolution in differentiating the overlap of the radiopaque markers (when viewed with an imaging technique through the stent graft) than might be available with thicker images, such as solidly shaded geometric shapes. Also, taking advantage of the amount of eclipsing or overlap between the oppositely positioned linear shapes may allow a better objective indication of rotational position of a stent graft device than a single radiopaque marker.

Other linear shapes are contemplated for the radiopaque markers 36, 38. For example, rather than two linear portions of different lengths connected by a bend as shown in the j-shaped or checkmark shaped version, the radiopaque markers may instead have the same length linear portions (line segments of equal length attached at an angle to form a symmetric linear shape) as in a "V" or "U" shape. Also, the oppositely mounted linear radiopaque markers may also have more than one bend in other implementations. For example, the linear radiopaque markers may form mirror-image serpentine shapes that each follow the stent frame element 12 along at least one sequential peak and valley of the stent frame element on opposite sides of the stent graft device 10.

As noted above, the first and second radiopaque markers may be directly attached to the same stent frame element 12 or to the graft portion 14. Depending on the design of the particular stent graft device 10, the stent element on which the radiopaque marker or markers is attached to may be on the inside or the outside of the tubular graft portion 14. The radiopaque markers 16,18 may be individually sewn onto the material of the tubular graft portion 14 and not attached to the stent frame elements 12 so that folding of the stent graft 10 is not affected. Alternatively, the radiopaque markers 16, 18 may be attached directly to the stent frame elements 12 where the stent graft 10 may not fold as neatly as described above. The radiopaque wire or thread used maybe a metal or non-metal radiopaque material, thread/suture (e.g. a gold thread or polypropylene impregnated with barium). Attachment to the tubular graft portion 14 may be implemented in the form of weaving the radiopaque material into the graft material using a thread having the gold or radiopaque substance incorporated into the thread itself. Alternatively, the radiopaque material may be retained along the inner or outer circumference of the graft inside a pocket sized to receive and retain the radiopaque material. The pocket may be made out the same material as the tubular graft portion land adhered or sewn shut to retain the radiopaque material in the desired position on the stent graft 10.

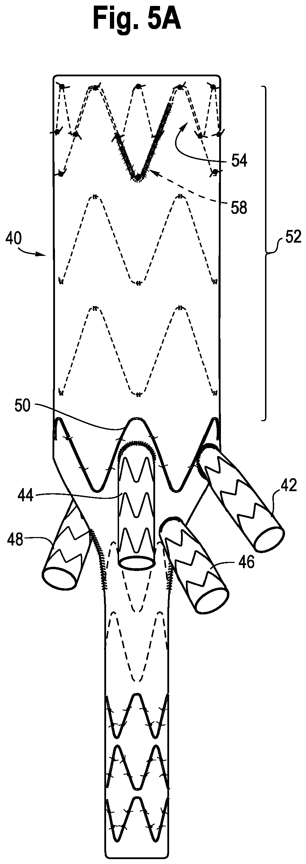

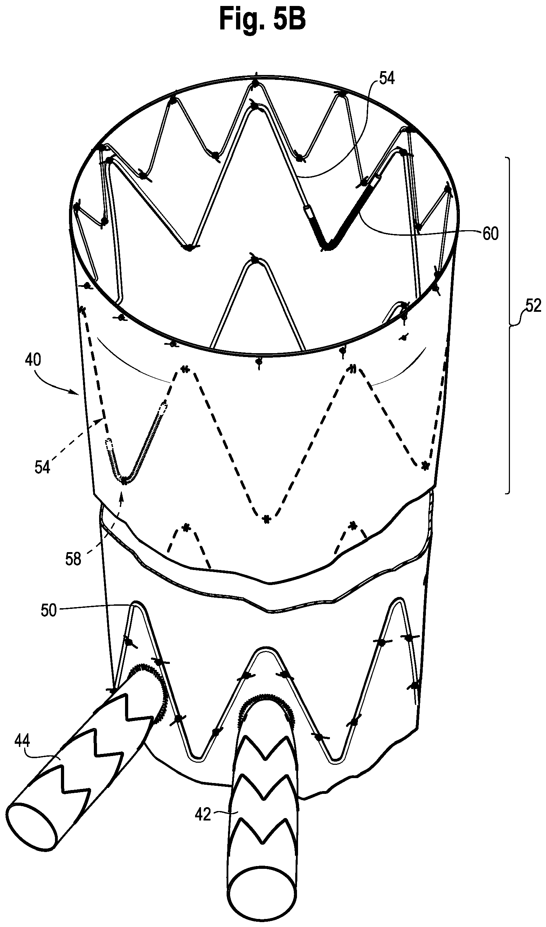

The stent graft 10 of FIGS. 1-4 represents a simplified tubular section of a stent graft 10 for ease of illustration, however any of a number of stent graft devices may utilize the double radiopaque marker arrangement described herein. Referring to FIG. 5, a thoracoabdominal aortic aneurism (TAAA) stent graft 40 is shown with four directional branches attached to a main aortic vessel body: a celiac artery branch 42, a SMA branch 44, a right renal artery (RRA) branch 46, and a left renal artery (LRA) branch 48. Device rotational angle accuracy for the TAAA stent graft 40 is necessary in order to align these four visceral branches to the respective target vessels (renal, celiac and SMA). Given that the visceral arterial diameters for each of the branches may typically be in the range of 5 mm - 8 mm, a higher accuracy rotational alignment system may improve results (e.g. time and accuracy) for medical practitioners and stent graft device recipients.

In the example of FIG. 5, the TAAA stent graft 40 may include mirror image j- shaped or checkmark shaped radiopaque markers 58, 60 positioned on opposite sides of a stent frame element 54 mounted internally to the tubular graft portion 56 in the upper region 52. The stent graft 40 may also include one or more externally mounted stent frame elements 50. As the internally mounted stent frame element 54 and first radiopaque marker 58 are inside the tubular graft portion, a dashed line representation of these two components is provided for ease of illustration in FIGS. 5A and 5B. The second radiopaque marker 60 (FIG. 5B) is positioned on the opposite side of the internal stent frame element 54 and, as in the example of FIGS. 1-2, is the mirror image, as viewed from the outside of the stent graft 40. Thus, an image from an imaging device looking through the stent graft 40 would show the first and second radiopaque markers 58, 60 in the same orientation and at a visually discernible overlap or rotational offset depending on the current rotational position of the stent graft about the longitudinal axis of the stent graft 40.

As has been described above, the use of two linear (e.g. checkmark or j-shaped) radiopaque markers that are positioned on opposite sides along a circumference of a stent graft and are mirror images of one another as viewed from their respective sides of the stent graft, allows for fine granularity imaging projections of the rotational position of the stent graft device. This may permit a user to place the stent graft in a desired rotational orientation before expanding the stent in the final location where it will be installed and thus may reduce guess work and inaccuracy. The asymmetric and oppositely mounted radiopaque markers may allow for relatively easy visualization via x-ray where the radiopaque markers will completely eclipse one another only if the stent graft is at a 6 o'clock or a 12 o'clock position and where a front or back view of the stent graft may be determined by the orientation of the radiopaque markers (e.g., left or right facing j or checkmark in one embodiment).

In summary, a stent graft device having radiopaque markers for rotational orientation. In one embodiment, the stent graft device may include an expandable stent frame and attached graft member. Two radiopaque markers, one the mirror image of the other, positioned on opposite sides of the stent graft at the same axial position along the longitudinal axis of the stent graft device, may be used to provide fine granularity rotational orientation of the stent graft. The two radiopaque markers may be attached to a same stent element and be in the form of checkmarks or some other linear asymmetric design that allow a user to view rotational orientation of the stent graft device by the amount of alignment and overlap of the two radiopaque markers when viewed via an imaging device such as an x-ray.

While various embodiments of the invention have been described, it will be apparent to those of ordinary skill in the art that many more embodiments and implementations are possible within the scope of the invention. Accordingly, the invention is not to be restricted except in light of the attached claims and their equivalents. Moreover, the advantages described herein are not necessarily the only advantages of the invention and it is not necessarily expected that every embodiment of the invention will achieve all of the advantages described.

The foregoing description of the inventions has been presented for purposes of illustration and description, and is not intended to be exhaustive or to limit the inventions to the precise forms disclosed. It will be apparent to those skilled in the art that the present inventions are susceptible of many variations and modifications coming within the scope of the following claims.

## Claims

1. A stent graft device comprising:
a stent frame having a central axis;
a graft member attached to the stent frame, wherein the stent graft device has a compressed state and an expanded state, wherein a diameter of the stent graft in the expanded state is greater than that of the stent graft device in the compressed state;
a first radiopaque marker positioned on a first longitudinally extending side of the stent graft device;
a second radiopaque marker positioned on a second longitudinally extending side of the stent graft device that is on an opposite side of the stent graft device from the first longitudinally extending side, the second radiopaque marker positioned at a same axial position along the central axis of the stent graft device as the first radiopaque marker, wherein the second radiopaque marker, as viewed from the second longitudinally extending side, comprises a mirror image of the first radiopaque marker as viewed from the first longitudinally extending side; and
whereby a unique rotational position of the stent graft device is detectable via a spacing or eclipsing of the first and second radiopaque markers in an image of the stent graft device.

2. The stent graft device of claim 1, wherein the first radiopaque marker is an asymmetrical line pattern, optionally wherein the asymmetrical line pattern comprises a checkmark shape.

3. The stent graft device of claim 1 or 2, wherein at least one of the first and second radiopaque markers is attached to the stent frame, optionally wherein at least one of the first and second radiopaque markers comprises a radiopaque wire wound around a portion of the stent frame.

4. The stent graft device of claim 1 or 2, wherein at least one of the first and second radiopaque markers is attached to the graft member.

5. A stent graft device comprising:
a plurality of stent frame elements arranged along a central axis;
a graft member attached with the stent frame elements, wherein the stent graft device has a compressed state and an expanded state, wherein a diameter of the stent graft device in the expanded state is greater than that of the stent graft device in the compressed state;
a first radiopaque marker having an asymmetric linear shape and positioned along a circumference of the stent graft device on a first longitudinally extending side of the stent graft device; and
a second radiopaque marker comprising a mirror image of the asymmetric linear shape of the first radiopaque marker, wherein the second radiopaque marker is on a second longitudinally extending side of the stent graft device, circumferentially offset 180 degrees along the circumference from the first radiopaque marker, and is at a same axial location along the central axis as the first radiopaque marker on the stent graft device; and
whereby a unique rotational position of the stent graft device is detectable via a spacing of the first and second radiopaque markers in an image of the stent graft device.

6. The stent graft device of claim 5, wherein at least one of the first and second radiopaque markers is attached to the graft member.

7. The stent graft device of claim 5 or 6, wherein the asymmetric linear shape comprises a checkmark shape.

8. The stent graft device of any of claims 5 to 7, wherein at least one of the first and second radiopaque markers is attached to one of the stent frame elements, optionally wherein both of the first and second radiopaque markers are attached to a same stent frame element.

9. The stent graft device of claim 8, wherein the at least one of the first and second radiopaque markers attached to the one of the stent frame elements comprises a radiopaque material attached to a portion of the one of the stent frame elements, optionally wherein the at least one of the first and second radiopaque markers attached to the one of the stent frame elements comprises a wire wound around the portion of the one of the stent frame elements.

10. A stent graft device comprising:
a radially expandable stent frame having a central axis;
a tubular graft member attached with a surface of the stent frame, wherein the stent graft device has a compressed state and an expanded state, and wherein a diameter of the stent graft device in the expanded state is greater than that of the stent graft device in the compressed state;
first and second radiopaque markers each a comprising a linear pattern having at least one bend, the first radiopaque marker is fixedly positioned on a first longitudinally extending side of the stent graft device and the second radiopaque marker is fixedly positioned on a second longitudinally extending side of the stent graft device on an opposite side of the stent graft device, and at a same axial position along the stent graft device, as the first radiopaque marker;
wherein an orientation of the first radiopaque marker when the first radiopaque marker is viewed from the first longitudinally extending side of the stent graft device is a mirror image of an orientation of the second radiopaque marker when the second radiopaque marker is viewed from the second longitudinally extending side of the stent graft device;
wherein the orientation of the first radiopaque marker is a same orientation as the orientation of the second radiopaque marker in an image generated by an imaging system through the stent graft device; and
whereby a unique rotational position of the stent graft device is detectable via a location of the first and second radiopaque markers in the image generated by the imaging system.

11. The stent graft device of claim 10, wherein at least one of the first or second radiopaque markers comprises a radiopaque material wound around a portion of a stent frame element of the stent frame.

12. The stent graft device of claim 10 or 11, wherein the imaging system is an X-ray system.

13. The stent graft device of any of claims 10 to 12, wherein at least one of the first and second radiopaque markers is attached to the tubular graft member.

14. The stent graft device of any of claims 10 to 13 , wherein the linear pattern having at least one bend comprises:
a first line segment attached at an angle to a second line segment by the bend, optionally wherein the first and second line segments are different lengths.

15. The stent graft device of any of claims 10 to 14, wherein the first and second radiopaque markers have identical lengths.
